# EUROPÄISCHE PATENTSCHRIFT

(11) **EP 1 980 196 B1**
(45) Veröffentlichungstag und Bekanntmachung des Hinweises auf die Patenterteilung: **06.04.2016**
(21) Anmeldenummer: 08154273.0
(22) Anmeldetag: 09.04.2008
(51) Int. Cl.: A61B 1/00, A61M 39/08, B29C 71/00, B29C 71/02, B29L 31/00

(54) **Endoskop mit einem Stülpschlauchantrieb**
Endoscope with everting tube arrangement
Endoscope avec tube de retournement

(30) Priorität: 10.04.2007 DE 102007000214
(43) Veröffentlichungstag der Anmeldung: 15.10.2008
(73) Patentinhaber: Invendo Medical GmbH, 69469 Weinheim (DE)
(72) Erfinder: Bob, Konstantin, 69469, Weinheim (DE)
(74) Vertreter: Winter, Brandl, Fürniss, Hübner, Röss, Kaiser, Polte - Partnerschaft mbB

(56) Entgegenhaltungen:
- EP-A2- 0 454 293
- WO-A1-89/09246
- WO-A1-99/24174
- DE-A1- 4 242 291
- DE-A1-102004 054 040
- JP-A- 2005 323 658
- US-A- 4 526 579
- US-A- 4 589 873
- US-A- 5 236 423
- US-A- 5 985 394
- US-A- 6 086 970
- US-A1- 2005 220 836
- US-A1- 2006 020 164
- US-B1- 6 610 068

## Beschreibung

### Technisches Gebiet

Die vorliegende Erfindung betrifft ein Endoskop mit einem Endoskopschaft und einem Stülpschlauch, durch deren Ausgestaltung das Einführen des Endoskops vereinfacht wird.

### Stand der Technik

Vorrichtungen zum Einführen eines medizinischen Endoskops in einen Körperkanal werden beispielsweise in der deutschen Patentanmeldung DE-A-39 25 484 beschrieben. Die darin beschriebenen Vorrichtungen erlauben es, dass ein Endoskop nicht mehr in den zu untersuchenden Körper hineingeschoben wird, sondern sich selber hineinbewegt. Zu diesem Zweck ist das Endoskop mit einem Eigenantrieb ausgestattet, der ein unkomplizierteres und schnelleres Einführen ermöglicht.

Als solch ein Eigenantrieb kann beispielsweise auch ein so genannter Stülpschlauch verwendet werden, in den der Endoskopschaft eingeführt ist. Beim Vortrieb des Endoskops treten unterschiedliche Relativbewegungen auf. Zum einen tritt eine Relativbewegung zwischen dem Endoskopschaft und dem Stülpschlauch auf, die miteinander in Gleitkontakt stehen. Zum anderen tritt auch eine Relativbewegung zwischen einem innenliegenden und einem außenliegenden Abschnitt des sich abwickelnden Stülpschlauchs auf.

Es gibt bei der gegenwärtigen Entwicklung derartiger Vorrichtungen das Bestreben, Schläuche herzustellen, die eine verminderte Reibung zwischen beispielsweise einem eingesetzten Stülpschlauch und einem Endoskopschaft, zwischen dem Stülpschlauch und einer Hülle des Endoskopschafts als auch zwischen einzelnen Abschnitten eines Stülpschlauchs erzielen können.

Werden beispielsweise Siliconschläuche eingesetzt, wird durch Bildung von Spaltprodukten bei der Herstellung leicht eine Klebrigkeit der Oberfläche, so genannter "tag", beobachtet. Dieser steht der angestrebten verminderten Reibung entgegen, sodass Siliconschläuche bislang nur zögerlich Verwendung bei medizintechnischen Vorrichtungen gefunden haben, die geringe Reibungen erfordern, wie etwa insbesondere in endoskopischen Anwendungen.

Aus dem Stand der Technik sind vielfältige Ansätze bekannt, das Einführen medizinischer Vorrichtung in den zu untersuchenden Körper zu vereinfachen. Diese umfassen Verfahren zur Behandlung von Oberflächen medizinischer Schläuche als auch Vorschläge für konstruktive Ausgestaltungen beispielsweise von Endoskopen. Beispielsweise offenbart die WO 99/24174 ein Verfahren, bei dem eine Oberfläche mit einer ersten Lösung überzogen wird, welche danach durch Behandlung mit beispielsweise Infrarotstrahlung zu einer ersten Beschichtung ausgeformt wird, wonach eine zweite Lösung, welche Zellulose enthält, aufgebracht wird, welche danach durch Erhitzen getrocknet wird.

Gemäß der WO 89 / 09246 wird eine Oberfläche zur Verringerung der Reibung mit einem kreuzvernetzten hydrophilen Polymer beschichtet.

Die Druckschrift US 4, 589, 873 offenbart weiterhin ein Verfahren zur Beschichtung einer Oberfläche mit Polyvinylpyrrolidon zur Verbesserung der Schmierfähigkeit. Weiterhin ist in der JP 2005-323658 ein Katheter offenbart mit einem inneren Schlauch mit hoher Gleitfähigkeit, welcher aus Polytetrafluororethylen gefertigt ist.

Aus der EP 0454 293 A2 ist weiterhin ein Verfahren bekannt zur Beschichtung einer Oberfläche mit einem Polyurethan zur Verringerung der Reibung.

Die Druckschrift US 4,526, 579 offenbart zudem ein Verfahren zur Beschichtung einer Oberfläche mit einer Carbonsäure oder einem Salz davon.

Zudem ist aus der US 5, 985, 394 ein Verfahren zur Herstellung eines Elastomers bekannt, welches einen hohen Anteil an Wasser enthält, was die Schmierfähigkeit verbessert.

Die Druckschrift US 2005 / 0220836 A1 offenbart weiterhin die Behandlung von Oberflächen medizinischer Schläuche mit medizinischen Wirkstoffen und Trägermaterialien, welche den Oberflächen der Schläuche vorteilhafte Eigenschaften zur Vereinfachung der Implantation der Schläuche und zur Verringerung der Wahrscheinlichkeit einer Abstoßung der Schläuche verleihen.

Aus der US 6, 610,068 B1 ist zudem ein Katheter mit einem Lumen für ein Führungsbauteil bekannt, bei dem das Lumen mit einem Gleitmittel gefüllt ist.

Ein weiteres Verfahren zur Oberflächenbehandlung ist aus der DE 102004054040A1 bekannt. Bei diesem Verfahren werden Silikonelastomere homogen mit antimikrobiell wirksamen Stoffen versetzt. Daraufhin werden aus den Silikonelastomeren Formkörper hergestellt.

Die Druckschrift US 6, 086,970 offenbart zudem einen Katheter mit verbesserter Gleitfähigkeit. Bei diesem Katheter weist die Oberfläche harte Partikel, wie z.B. Glaskügelchen, und ein Gleitmittel, wie z.B. ein Wachs, auf.

Aus der US 5, 236, 423 ist zudem ein Verfahren zum Einführen einer medizinischen Vorrichtung in eine Körperöffnung bekannt, bei dem zunächst ein erster Schlauch in die Körperöffnung eingeführt wird, durch den in einem zweiten Schnitt die medizinische Vorrichtung eingeführt wird.

Weiterhin offenbart die Druckschrift DE 4242291 A1 ein Verfahren zur Einführung eines Endoskops in ein Hohlorgan mittels eines Stülpschlauchs.

Zudem ist aus der Druckschrift US 2006 / 0020164 A1 ein weiteres endoskop mit einem Stülpschlauch bekannt.

### Darstellung der Erfindung

Die vorliegende Erfindung ist im Hinblick auf die genannten Bestrebungen gemacht worden. Dabei ist es eine Aufgabe der Erfindung, ein Endoskop bereitzustellen, das sich leicht einführen lässt. Die vorliegende Erfindung stellt hierfür ein Endoskop mit einem Stülpschlauchantrieb bereit, wobei das Endoskop (bzw. der Endoskopschaft oder eine darüber gestülpte Endoskopschafthülle) und/oder der Stülpschlauch aus einem Kautschukmaterial besteht und auf wenigstens einer der Oberflächen des Stülpschlauchs und/oder der Oberfläche des Endoskops noppenförmige Vorsprünge ausgebildet sind.

Zudem wird ein Verfahren zur Herstellung eines medizintechnischen Kautschukschlauchs zur Verwendung in einem erfindungsgemäßen Endoskop beschrieben, dessen Reibung und "tag" vermindert sind. Dabei sollte das Verfahren möglichst einfach sein, da ein derartiger Kautschukschlauch ggf. ein Einwegartikel ist und somit in der Herstellung kostengünstig sein sollte. Zudem sollte sich das Verfahren möglichst leicht an unterschiedliche Schlauchmaterialien anpassen lassen. Ferner ist es wünschenswert, dass das Verfahren einen Schlauch liefert, der sich mit weiteren Materialien wie etwa ergänzenden Schmiermitteln leicht verhaften bzw. beschichten lässt. Es ist eine weitere Aufgabe der Erfindung, einen entsprechenden Kautschukschlauch als solchen bereitzustellen, dessen Gleiteigenschaften wie oben beschrieben verbessert sind, der geringeres "tag" zeigt, kostengünstig ist und sich mit unterschiedlichen weiteren Schmiermitteln hervorragend kombinieren lässt.

Auch wird ein Verfahren zur Verringerung des Oberflächenreibungskoeffizienten (ja) eines medizintechnischen Kautschukschlauchs beschrieben, mit den Schritten: Herstellen eines Schlauchs aus Kautschukmaterial, und Nachbehandeln des Schlauchs durch Tempern und/oder Waschen.

Das Kautschukmaterial ist bevorzugt aus Siliconmaterial, PTFE-Material, ePTFE-Material und thermoplastischen Kunststoffen, vorzugsweise thermoplastischem Polyurethan, in welches vorzugsweise ein Öl eingearbeitet ist, ausgewählt. In einer bevorzugten Ausführungsform wird das Siliconmaterial beim Herstellen mit einer Platinverbindung additionsvernetzt.

In einer weiteren bevorzugten Ausführungsform wird das Siliconmaterial beim Herstellen peroxidisch vernetzt. Bevorzugt wird das Kautschukmaterial beim Herstellen zu einem Schlauch extrudiert. Dabei ist es insbesondere bevorzugt, dass entweder beim Extrusionsvorgang selber oder nach dem Extrusionsvorgang in einem gesonderten Schritt Noppen auf mindestens einer Schlauchoberfläche erzeugt werden.

Die beim Extrusionsvorgang erzeugte Noppe ist beispielsweise ein kontinuierlicher, spiralförmig umlaufender Vorsprung. Beim Nachbehandeln erfolgt das Tempern vorzugsweise bei 120 bis 180 deg. C. Beim Nachbehandeln wird vorzugsweise für 30 bis 180 Minuten getempert.

Zudem wird beim Nachbehandeln bevorzugt bei 30 bis 90 deg. C gewaschen. Beim Nachbehandeln wird außerdem bevorzugt für 30 bis 60 Minuten gewaschen.
Das Waschen erfolgt vorzugsweise mit wässriger, basischer Waschflüssigkeit.
In einer weiteren bevorzugten Ausführungsform wird der Schlauch im Anschluss an die Nachbehandlung mit einem Schmiermittel beschichtet.
Vorzugsweise ist dabei das Schmiermittel aus Agar-Agar, pflanzlichem Öl, einer Fett-Wachs-Mischung, (e)PTFE-Gleitlack, (e)PTFE-Pulver, Graphit, Talkum, weiteren, von dem Siliconmaterial des Schlauchs verschiedenen Siliconüberzügen, Siliconöl, in Schmieröl dispergiertem Graphit- und/oder Teflon-Pulver sowie in Schmieröl dispergierten (e)PTFE- und/oder Glaskügelchen oder einer Kombination von zwei oder mehreren von diesen ausgewählt.

In einer bevorzugten Ausführungsform wird die Oberfläche des Schlauchs im Anschluss an die Nachbehandlung (jedoch vor dem eventuellen Verfahrensschritt des Beschichtens mit Schmiermittel) mit (e)PTFE-Kugeln und/oder Glaskugeln besetzt. Insbesondere ist der Schlauch bei den beschriebenen Verfahren eine Schafthülle für ein erfindungsgemäßes Endoskop oder ein Stülpschlauch für ein erfindungsgemäßes Endoskop.
Der Stülpschlauch wickelt sich dabei auf der Endoskopschafthülle ab. Vorzugsweise ist das Kautschukmaterial aus Siliconmaterial, PTFE-Material, ePTFE-Material und thermoplastischen Kunststoffen, vorzugsweise thermoplastischem Polyurethan, in welches vorzugsweise ein Öl eingearbeitet ist, ausgewählt.

Dabei ist es des Weiteren bevorzugt, dass sich zwischen und/oder in den noppenförmigen Vorsprüngen ein wachshaltiges Fett oder ein Öl befindet.
Dabei ist es bevorzugt, dass die noppenförmigen Vorsprünge als perforierte Schmiermittelreservoire ausgebildet sind. In einer bevorzugten Ausführungsform sind die noppenförmigen Vorsprünge durch Kugeln aus (e)PTFE oder Glas gebildet, die an der jeweiligen Oberfläche verhaftet sind.

Der bevorzugte Kugeldurchmesser für die Kugeln liegt im Bereich von >0,1 bis <0,2 mm.

Demnach hat das Verfahren vorzugsweise den Verfahrensschritt, wobei beim Nachbehandeln für 30 bis 60 Minuten gewaschen wird. Weiter vorzugsweise ist der Verfahrensschritt vorgesehen, dass beim Nachbehandeln mit wässriger, basischer Waschflüssigkeit gewaschen wird. Weiter vorzugsweise ist der Verfahrensschritt vorgesehen, dass der Schlauch im Anschluss an die Nachbehandlung mit einem Schmiermittel beschichtet wird. Weiter vorzugsweise ist der Verfahrensschritt vorgesehen, dass das Schmiermittel aus Agar-Agar, pflanzlichem Öl, einer Fett-Wachs-Mischung, (e)PTFE-Gleitlack, (e)PTFE-Pulver, Graphit, Talkum, weiteren, von dem Siliconmaterial des Schlauchs verschiedenen Siliconüberzügen, Siliconöl, in Schmieröl dispergiertem Graphit- und/oder Teflon-Pulver sowie in Schmieröl dispergierten (e)PTFE und/oder Glaskügelchen oder einer Kombination von zwei oder mehreren von diesen ausgewählt ist. Weiter vorzugsweise ist der Verfahrensschritt vorgesehen, dass die Oberfläche des Schlauchs im Anschluss an die Nachbehandlung mit (e)PTFE-Kugeln und/oder Glaskugeln besetzt wird. Weiter vorzugsweise ist der Verfahrensschritt vorgesehen, dass der Schlauch eine Schafthülle für ein Endoskop oder ein Stülpschlauch für ein Endoskop ist. Weiter vorzugsweise ist es vorgesehen, dass ein Medizintechnischer Kautschukschlauch geschaffen wird, der durch ein Verfahren mit zumindest einem der vorstehenden Verfahrensschritte erhältlich ist.

Weiter vorzugsweise wird ein Endoskop mit einem Stülpschlauchantrieb geschaffen, wobei das Endoskop und/oder der Stülpschlauch aus einem Kautschukmaterial besteht, wobei auf wenigstens einer der Oberflächen des Stülpschlauchs und/oder der Oberfläche des Endoskops noppenförmige Vorsprünge ausgebildet sind. Weiter vorzugsweise ist es vorgesehen, das das Kautschukmaterial des Endoskops aus Siliconmaterial, PTFE-Material, ePTFE-Material und thermoplastischen Kunststoffen, vorzugsweise thermoplastischem Polyurethan, in welches vorzugsweise ein Öl eingearbeitet ist, ausgewählt ist. Weiter vorzugsweise ist es bezüglich des Endoskops vorgesehen, dass sich zwischen und/oder in den noppenförmigen Vorsprüngen ein wachshaltiges Fett oder ein Öl befindet.

Weiter vorzugsweise ist es bezüglich des Endoskops vorgesehen, dass die noppenförmigen Vorsprünge als perforierte Schmiermittelreservoire ausgebildet sind. Weiter vorzugsweise ist es bezüglich des Endoskops vorgesehen, dass die noppenförmigen Vorsprünge durch Kugeln aus (e)PTFE oder Glas gebildet sind, die an der jeweiligen Oberfläche verhaftet sind. Weiter vorzugsweise ist es bezüglich des Endoskops vorgesehen, dass die Kugeln einen Kugeldurchmesser im Bereich von >0,1 bis <0,2 mm aufweisen.

Ergänzende Aufgaben, Vorteile und bevorzugte Ausführungsformen sind der nachstehenden detaillierten Beschreibung der Erfindung zu entnehmen.

### Detaillierte Beschreibung der Erfindung

Das beschriebene Verfahren zur Verringerung des Oberflächen-Reibungskoeffizienten (ja) eines medizintechnischen Kautschukschlauchs erfordert zunächst einmal den Schritt des Herstellens eines Schlauches. Das Kautschukmaterial ist dabei bevorzugt aus Siliconmaterial, PTFE-Material, ePTFE-Material und thermoplastischen Kunststoffen, vorzugsweise thermoplastischem Polyurethan, in welches vorzugsweise ein Öl eingearbeitet ist, ausgewählt.

Das vorzugsweise zum Einsatz kommende Siliconmaterial ist, ebenso wie die anderen vorstehend genannten Kautschukarten, durch seine inerten Eigenschaften grundsätzlich für medizintechnische Anwendungen gut geeignet, allerdings ergeben sich bei der Polymerisation selber oder später durch Umwelteinflüsse, auch in Abhängigkeit von den an die Siliciumatome der Edukte gebunden Gruppen, unterschiedliche (Ab)Spaltprodukte wie z.B. Alkohole, Säuren, Derivate davon, Polymerfragmente, etc. Derartige Spaltprodukte führen oftmals zu erhöhter Haftung und folglich zu vergrößerter Reibung der hergestellten Schläuche. Auch können ggf. beim Herstellungsvorgang unerwünschte ölartige Rückstände abgesondert werden, die bei späterer Beschichtung, z.B. mit einem Schmiermittel, zu einer verringerten bzw. fehlenden Verhaftung zwischen Schlauch und Beschichtung führen können.

Eine erste Maßnahme zur Verringerung der Spaltproduktmenge besteht darin, das Siliconmaterial im Herstellungsvorgang einer Additionsvernetzung zu unterziehen, vorzugsweise einer Additionsvernetzung unter Einsatz von Platin-Verbindungen. Hierbei eignen sich insbesondere Pt(0)-Komplexe, die als Liganden z.B. Vinylsiloxane aufweise können.

Aufgrund der deutlich verringerten Erzeugung von Spaltprodukten durch die Additionsvernetzung wird die Reibung vorteilhaft herabgesetzt. Ein aus einem derartigen additionsvernetzten Siliconmaterial hergestellter Schlauch eignet sich insbesondere gut als Stülpschlauch, welcher eingesetzt wird, um eine darin eingeführte Vorrichtung, etwa den Schaft (mit oder ohne Hülle) eines erfindungsgemäßen Endoskops, vorwärts zu treiben.

Eine weitere vorteilhafte Maßnahme zur Verringerung der Spaltproduktmenge besteht darin, das Siliconmaterial beim Herstellungsvorgang peroxidisch zu vernetzen. Bei einer peroxidischen Vernetzung können beispielsweise Alkyl- oder Arylperoxide wie z.B. Dicumylperoxid, 1 ,4-Bis(tert-butylperoxy)-1 ,4-dimethylhexan, 2,4-Dichlorbenzoylperoxid und 4-Methylbenzoylperoxid zum Einsatz kommen. Zwar liefert eine peroxidische Vernetzung geringfügig grössere Mengen an Spaltprodukt als eine Additionsvernetzung, allerdings ist deren Anteil gegenüber einem nicht vernetzten Siliconmaterial immer noch stark verbessert. Darüber hinaus ist die peroxidische Vernetzung äußerst kostengünstig.

Aus diesem Grund wird die peroxidische Vernetzung bevorzugt in dem Fall eingesetzt, dass der herzustellende Schlauch ein Einwegartikel ist, beispielsweise eine über dem Schaft eines erfindungsgemäßen Endoskops anzuordnende Hülle (Schafthülle).

Insbesondere wenn der medizintechnische Kautschukschlauch als Stülpschlauch eingesetzt wird, eignet sich Siliconmaterial besonders gut als Grundsubstanz für diesen Schlauch, weil es vergleichsweise kostengünstig ist und leicht so bearbeitet werden kann, dass die angestrebte Verringerung von i gerade auch in den Umstülpbereichen erzielt werden kann.

Kommt der medizintechnische Kautschukschlauch als Schafthülle zum Einsatz, ist auch ein thermoplastischer Kautschuk sehr gut geeignet, vorzugsweise ein thermoplastisches Polyurethan. Hiermit lässt sich eine gute Verbindung mit dem eigentlichen Schaft bei gleichzeitiger Reibungsverringerung nach Außen erzielen. Die Ausformung des Kautschukmaterials in Schlauchform kann durch an sich bekannte Verfahren der Kautschukverarbeitung erfolgen. Bevorzugt wird erfindungsgemäß ein Extrusionsvorgang, da hierbei dem Schlauch eventuell erwünschte spezielle Querschnittsgestalten und/oder Profile verliehen werden können. Vorteilhafter Weise werden bei einem derartigen Extrusionsvorgang noppenförmige Vorsprünge auf wenigstens einer Schlauchoberfläche erzeugt. "Schlauchoberfläche" meint hierbei sowohl die Außen- als auch Innen(mantel)fläche des Schlauchs.

Beim Extrudieren kann dabei besonders gut eine auf der Schlauchoberfläche durchgängige, spiralförmig umlaufende Noppe (Vorsprung) ausgebildet werden.

Es ist ferner möglich, die Noppen in einem der Extrusion nachfolgenden gesonderten Schritt zu erzeugen, beispielsweise durch Schneiden, Stanzen, Prägen, Lasergravur oder dergleichen. Bei diesen Vorgängen wird in der Regel das sich zwischen den Noppen verbleibende Material abgetragen. Die Noppen können allerdings in dem gesonderten Schritt auch aus einer glatten Oberfläche heraus erzeugt werden, beispielsweise durch Aufschäumen spezieller Oberflächenbereiche, etwa durch lokales, z.B. punktförmiges, Erhitzen der Oberfläche (beispielsweise mit einem Laser).

Derartige noppenförmige Vorsprünge (bzw. Noppen), z.B. eine spiralförmige Noppe, auf der Oberfläche dienen als Abstandhalter auf der Schlauchoberfläche, zwischen welche ein Schmiermittel eingefüllt werden kann.

Möglich ist auch, dass sich das Schmiermittel (wachshaltiges Fett, Öl wie etwa Siliconöl, Pflanzenfett oder -öl) alternativ oder ergänzend in den Noppen selber befindet, welche dann als Schmiermittelreservoir dienen. Aus diesen Schmiermittelreservoiren kann das Schmiermittel beispielsweise bei Druckbeaufschlagung austreten. Der Austritt des Schmiermittels kann weiterhin vereinfacht werden, indem die Schmiermittelreservoire perforiert sind. Die Perforation kann gleichzeitig mit der Noppenerzeugung oder in einem weiteren Verfahrensschritt erfolgen.

In beiden Fällen, d.h. Schmiermittel zwischen bzw. in den Noppen, wird die Gleitfähigkeit der Oberfläche nochmals deutlich verbessert. Das Schmiermittel kann dabei auf die so hergestellte Noppenfläche aufgebracht werden, oder nachdem die Noppenfläche mit einem Gleitlack, z.B. einem (e)PTFE-Gleitlack, überzogen oder plasmabehandelt bzw. plasmabeschichtet wurde. Das Schmiermittel wird dabei in Abhängigkeit von seinen chemischen und physikalischen Eigenschaften vor oder nach der später beschriebenen Nachbehandlung des beschriebenen Verfahrens aufgebracht. Geeignete Schmiermittel werden später beschrieben.

Bei demVerfahren erfolgt nach der Herstellung des Schlauchs aus Kautschukmaterial eine Nachbehandlung des Schlauchs durch Tempern und/oder Waschen. Durch beide Nachbehandlungen kann die Menge an noch auf der Oberfläche vorliegenden Spaltprodukten und öligen Rückständen deutlich vermindert werden, was somit jeweils zu einer Verbesserung der Gleiteigenschaften bzw. besserer Verhaftung mit einer später aufgebrachten Beschichtung führt. Die besten Ergebnisse werden erzielt, wenn beide Nachbehandlungen in beliebiger Reihenfolge durchgeführt werden. Falls erforderlich kann der Kautschuk zudem vulkanisiert werden, wobei typische Vulkanisationsmittel, z.B. Schwefel, schwefelhaltige Verbindungen, Peroxide und Zinkverbindungen wie Zinkweiss, zum Einsatz kommen.

Im Falle des Temperns erfolgt dieses vorzugsweise bei 120 bis 180 deg. C. Bei niedrigeren Temperaturen werden die Spaltprodukte/öligen Rückstände manchmal nur unzureichend entfernt, während höhere Temperaturen keine weiteren Verbesserungen liefern und in einigen Fällen das Kautschukgefüge schädigen können.

Ferner ist es im Falle des Temperns bevorzugt, für 30 bis 180 Minuten zu tempern. Bei kürzeren Temperzeiten besteht die Möglichkeit, dass die Spaltprodukte/öligen Rückstände nur unzureichend entfernt werden, während längere Temperzeiten meist keine weitere Verringerung des "tag" ergeben und dann unwirtschaftlich sind.

Die Temperaturen beim Tempern sowie die Temperzeiten werden bevorzugt in Abhängigkeit von der eingesetzten Vernetzungsmethode ausgewählt. Insbesondere bei additionsvernetzten Siliconmaterialien kann dadurch die Drucksteifigkeit des erhaltenen Siliconschlauchs verbessert werden, was insbesondere bei der Verwendung desselben als Stülpschlauch von Vorteil ist. Hierzu liegt die bevorzugte Tempertemperatur im Bereich von 120 bis 140 deg. C, für die Temperzeit ist der Bereich von 30 bis 60 Minuten vorzuziehen. Die besten Ergebnisse werden erzielt, wenn Temperzeit und Tempertemperatur gleichzeitig aus den genannten Bereichen ausgewählt sind. Eine signifikante Verbesserung der Drucksteifigkeit des Schlauchs ergibt sich etwa für die bevorzugte Kombination 140 deg. C130 Minuten.

Andererseits werden die beschriebenen Vorteile des Tempervorgangs, beispielsweise bei peroxidisch vernetzten Siliconmaterialien, in verlässlicher Weise erzielt, wenn etwas höhere Tempertemperaturen von bevorzugt 160 bis 180 deg. C zum Einsatz kommen. Aus demselben Grund wird vorzugsweise für 150 bis 180 Minuten getempert. Die besten Resultate ergeben sich wiederum, wenn beide bevorzugten Bereiche gleichzeitig eingehalten werden, beispielsweise mit der Kombination 180 deg. C/180 Minuten.

Im Falle des Waschens wird vorzugsweise bei 30 bis 90 deg. C gewaschen. Bei niedrigeren Temperaturen werden die Spaltprodukte/öligen Rückstände bisweilen nicht angemessen entfernt, während höhere Temperaturen oft keine bessere Wirkung zeigen und nur höhere Kosten erzeugen.

Zudem wird im Falle des Waschens bevorzugt für 30 bis 60 Minuten gewaschen. Bei kürzeren Waschzeiten ist die Entfernung der Spaltprodukte/öligen Rückstände leicht unzureichend, während längere Waschzeiten meist keine weitere Wirkung zeigen.

Der Waschvorgang kann ganz einfach in einer handelsüblichen Waschmaschine erfolgen. Als Waschflüssigkeit kommt vorzugsweise eine wässrige Waschflüssigkeit zum Einsatz, vorzugsweise eine leicht basische Waschflüssigkeit (pH >7 und <9). Bereits mit handelsüblichen Waschmitteln für Bekleidung können sehr gute Wirkungen erzielt werden. In einem konkret durchgeführten Test wurde ein erfindungsgemäß hergestellter Silikonschlauch in eine Waschmaschine gegeben und mit Wasser gewaschen, wobei 30 g NaCH zugesetzt wurden. Der Waschvorgang wurde bei 60 deg. C für 45 Minuten durchgeführt. Nach Beendigung des Waschvorgangs wurde 15 Minuten gewartet, danach wurde der gewaschene Schlauch mit destilliertem Wasser bis zur Neutralität gewaschen.

Der durch das vorstehend beschriebene Verfahren hergestellte Schlauch ist bereits als solcher sehr gut für medizintechnische Anwendungen, insbesondere in der Endoskopie, geeignet. Um eine weitere Verbesserung der Gleiteigenschaften zu erreichen, ist oftmals von Vorteil, ein weiteres Schmiermittel auf die einer Reibung ausgesetzten Schlauchoberflächen aufzubringen. Besonders verträgliche und am Kautschuk gut haftende Schmiermittel sind Agar-Agar, (e)PTFE-Gleitlack, Talkum, Graphit, weitere, von dem Siliconmaterial des Schlauchs verschiedene Siliconüberzüge (beispielsweise durch Plasmabeschichtung aufgetragen), Siliconöl, in Schmieröl dispergiertes Graphit- und/oder Teflon-Pulver sowie in Schmieröl dispergierte (e)PTFE-und/oder Glaskügelchen oder eine Kombination von zwei oder mehreren der aufgeführten Schmiermitteln.

Anstatt eines (e)PTFE-Gleitlacks kann auch ein (e)PTFE-Pulver eingesetzt werden (Nanopartikel). Dieses Pulver wird vorzugsweise durch Plasmabeschichtung aufgetragen. Um die Haftung am Schlauch zu verbessern, kann dieser vor dem Auftragen des Pulvers geätzt werden, vorzugsweise mittels Plasmaätzen.

Die gute Haftung des zusätzlichen Schmiermittels am Schlauch wird dabei überraschenderweise durch eine Vorbehandlung (Tempern und/oder Waschen) erzielt, d.h. es wird nicht nur der "tag" des Schlauches verringert, sondern zudem auch noch seine Kompatibilität gegenüber anderen Gleitmitteln verbessert. Beispielsweise wurde für die Haftung von Agar-Agar gefunden, dass diese sich gegenüber einem nicht nachbehandelten Schlauch (= 100%) nach Tempern als auch Waschen auf bis zu 150% verbessern ließ.

Das erfindungsgemäße Endoskop weist einen Stülpschlauchantrieb auf, wobei das Endoskop (d.h. der Endoskopschaft oder eine darüber gestülpte Endoskopschafthülle) und/oder der Stülpschlauch aus einem Kautschukmaterial besteht, welches vorzugsweise aus den oben genannten ausgewählt ist. Zur Verbesserung der Gleiteigenschaften sind auf wenigstens einer der Oberflächen des Stülpschlauchs und/oder der Oberfläche des Endoskops (Schaft oder Schafthülle) noppenförmige Vorsprünge ausgebildet. Es hat sich gezeigt, dass eine derartige Struktur zu einer deutlichen Verringerung von i führt.

Spezielle Ausgestaltungen der noppenförmige Vorsprünge sind die oben genannten Noppen, die beispielsweise direkt bei einem Extrusionsvorgang oder in einem nachfolgenden, separaten Verfahrensschritt erzeugt werden können, als auch Kugeln aus (e)PTFE oder Glas, die an der jeweiligen Oberfläche verhaftet sind. D.h., die Kugeln gleiten auf der gegenüberliegenden Gleitfläche, bewegen sich aber auf dem Schlauch selber im Wesentlichen nicht.

Vorteilhafte Kugeldurchmesser liegen im Bereich von >0,1 bis <0,2 mm. In diesem Bereich ist die Handhabung bei der Auftragung hinreichend einfach und es ergibt sich zudem die optimale Schmierwirkung.

Der Einsatz derartiger Kugeln bewirkt eine weitergehende Verringerung der Reibung, welche sowohl bei einer Reibung Schlauch-auf-Schlauch als auch bei einer Reibung Schlauch-auf-Schaft deutlich verbesserte Gleiteigenschaften ergibt. Ein Grund für diese Verbesserung liegt in einer deutlich verkleinerten Auflagefläche, nämlich nur dem tangentialen Bereich der Kugeln.

Zudem dienen die Kugeln als Abstandshalter, zwischen welche ein weiteres Schmiermittel eingefüllt werden kann. Die angehafteten Kugeln entsprechen diesbezüglich in ihrer Wirkung den oben erwähnten Noppen, d.h. diese beiden Ausführungsformen können als gleichwirkende und dem gleichen Konzept folgende Alternativen der noppenförmigen Vorsprünge angesehen werden.

Als Schmiermittel kommen in beiden Ausführungsformen die oben genannten Schmiermittel sowie insbesondere nichthydrierte oder teilweise bis vollständig hydrierte pflanzliche Öle wie etwa Rapsöl und Sonnenblumenöl in Betracht.

Besonders gut ist auch ein wachshaltiges Fett bzw. Fettgemisch geeignet, etwa ein Öl oder Ölgemisch, vorzugsweise eine Mischung aus pflanzlichem Öl und Wachs.

Es hat sich gezeigt, dass der Einsatz einer 50:50-Mischung aus vollständig hydriertem Rapsöl und hoch ölsäurehaltigem Sonnenblumenöl (so genanntes HO-Sonnenblumenöl), die einen Erstarrungspunkt im Bereich von bevorzugt 25 bis 50 deg. C, mehr bevorzugt 30 bis 40 deg. C und insbesondere bevorzugt 35 bis 39 deg. C sowie einen Klarschmelzpunkt von bevorzugt 50 bis 60 und mehr bevorzugt 54 bis 56 deg. C aufweist, sehr gute Gleiteigenschaften hat und in endoskopischen Anwendungen mit guter Verträglichkeit eingesetzt werden kann.

Um die Haftung einer derartigen Mischung an dem Kautschukschlauch (entweder dem Kautschukschlauch als solchem oder dem Kautschukschlauch mit noppenförmigen Vorsprüngen) noch weiter zu verbessern, ist der Mischung vorzugsweise eine gewisse Menge an Wachs beigefügt bzw. bereits enthalten, etwa in nicht-winterisiertem Öl (nichtfraktioniertem Öl).

Ein oben beschriebenes Fett, Fettgemisch, Öl bzw. Ölgemisch kann auch in das Kautschukmaterial selber eingebracht sein, vorzugsweise in das Kautschukmaterial aus thermoplastischem Kautschuk, insbesondere jenem aus thermoplastischem Polyurethan.

Wie vorstehend erwähnt ist das Verfahren insbesondere darauf ausgerichtet, einen Kautschukschlauch herzustellen, der eine Schafthülle für ein erfindungsgemäßes Endoskop oder einen Stülpschlauch für ein erfindungsgemäßes Endoskop bildet.

Als besonders geeignet haben sich in Versuchen mit tatsächlicher Endoskopie an Probanden die zwei nachstehend beschriebenen Arten von Siliconschläuchen erwiesen.

Zum einen ließ sich am Patienten die Endoskopie verglichen mit einem herkömmlichen Gerät viel einfacher (d.h. schneller, einfacher in der Handhabung und deutlich angenehmer für den untersuchten Patienten) durch Einsatz eines Stülpschlauches als Vortriebsmittel vornehmen, für welchen ein medizintechnischer Siliconschlauch eingesetzt wurde, der auf wenigstens einer seiner Oberflächen, im konkreten Fall der äußeren Oberfläche, Noppen aufwies.

In einem ergänzenden Versuch wurde gefunden, dass ein Siliconschlauch, bei dem sich zwischen den Noppen ein wachshaltiges Fett befindet (die oben genannte 50:50-Mischung aus Rapsöl und Sonnenblumenöl mit einem Erstarrungspunkt von ca. 37 deg. C und einem Klarschmelzpunkt von ca. 55 deg. C), bessere Gleiteigenschaften aufwies als einer ohne dieses Fett.

In ähnlicher Weise wurde in einer zweiten Studie ein herkömmliches Endoskop mit einem erfindungsgemäßen Endoskop verglichen, für welches ein Stülpschlauch zum Einsatz kam, der aus einem Kautschukschlauch (hier aus Silicon) mit Kugeln auf seiner äußeren Oberfläche bestand. Für das mit dem diesem Schlauch betriebene erfindungsgemäße Endoskop ergaben sich wiederum die Vorteile der schnelleren Untersuchung, der sichereren Handhabung durch den Untersuchenden sowie eines besseren Gefühls beim Patienten. Diese Vorteile konnten für Kugeln sowohl aus PTFE, ePTFE als auch Glas erzielt werden.

Versuche mit unterschiedlichen Kugeldurchmessern lieferten die besten Gleiteigenschaften für Kugeldurchmesser im Bereich von 0,1 bis 0,2 mm.

Die Verwendung der vorstehend beschriebenen Kautschukschläuche als Schafthülle für ein erfindungsgemäßes Endoskop oder als Stülpschlauch für ein erfindungsgemäßes Endoskop ist aufgrund der hervorragenden Gleiteigenschaften, der Kompatibilität mit verschiedenen Schmiermitteln, ihrer geringen Kosten sowie der leichten Herstellbarkeit besonders vorteilhaft und eine derartige Verwendung ist ebenfalls in die Erfindung eingeschlossen.

### Zusammenfassung

Die vorliegende Erfindung betrifft ein Endoskop mit einem Endoskopschaft und einem Stülpschlauch, durch deren Ausgestaltung das Einführen des Endoskops vereinfacht wird.

## Patentansprüche

1. Endoskop mit einem Endoskopschaft, der aus einem extrudierten Silikonmaterial gefertigt ist, und mit einem Stülpschlauchantrieb bestehend aus einem Stülpschlauch, der den Endoskopschaft zur Übertragung einer Vortriebskraft gleitfähig relativ beweglich umgibt, wobei der Stülpschlauch aus einem extrudierten Kautschukmaterial besteht, wobei der Stülpschlauch durch Tempern und/oder Waschen nachbehandelt ist, **dadurch gekennzeichnet dass** der Endoskopschaft an seiner Umfangsoberfläche eine durchgängige, spiralförmig umlaufende Vorsprungsnoppe aufweist, die bei der Extrusion des Endoskopschafts erzeugt worden ist, und die als Abstandshalter zwischen dem Endoskopschaft und dem diesen umgebenden Stülpschlauch einen Abstand definiert, in den ein Schmiermittel eingebracht ist, wobei das Schmiermittel auch in die Noppenfläche der Vorsprungsnoppe eingearbeitet ist, um bei einer Druckbeaufschlagung aus dieser auszutreten.

2. Endoskop nach Anspruch 1, wobei das Kautschukmaterial aus Silikonmaterial, PTFE-Material, ePTFE-Material und thermoplastischen Kunststoffen, vorzugsweise thermoplastischem Polyurethan, in welches vorzugsweise ein Öl eingearbeitet ist, ausgewählt ist.

3. Endoskop nach Anspruch 2, wobei das Silikonmaterial mit einer Platinverbindung additionsvernetzt ist.

4. Endoskop nach Anspruch 2, wobei das Silikonmaterial peroxidisch vernetzt ist.

5. Endoskop nach einem der Ansprüche 1 bis 4, wobei das Kautschukmaterial beim Herstellen zu einem Schlauch extrudiert ist.

6. Endoskop nach einem der Ansprüche 1 bis 5, wobei der Stülpschlauch durch Tempern bei 120 bis 180 deg. C nachbehandelt ist.

7. Endoskop nach einem der Ansprüche 1 bis 6, wobei der Stülpschlauch durch Tempern für 30 bis 180 Minuten nachbehandelt ist.

8. Endoskop nach einem der Ansprüche 1 bis 7, wobei der Stülpschlauch durch Waschen bei 30 bis 90 deg. C nachbehandelt ist.

## Claims

1. Endoscope having an endoscope shaft which is made from an extruded silicon material, and an everting tube arrangement consisting of an everting tube which with relatively sliding moveability surrounds the endoscope shaft for transmitting a driving force, wherein the everting tube is made from an extruded rubber material, wherein the everting tube is post-treated by heat-treatment and/or washing, **characterized in that** the endoscope shaft comprises a continuous, spiral-shaped and circumferential projection knob on its peripheral surface, which has been created by the extrusion of the endoscope shaft, and which, as a spacer between the endoscope shaft and the everting tube surrounding it, defines a space in which a lubricant is introduced, wherein the lubricant is incorporated also in the knob surface of the projection knob, so as to exit from it upon pressure application.

2. Endoscope according to claim 1, wherein the rubber material is selected from silicon material, PTFE material, ePTFE material and thermoplastic materials, preferably thermoplastic polyurethane, in which preferably an oil is incorporated.

3. Endoscope according to claim 2, wherein the silicon material is addition cross-linked with a platinum compound.

4. Endoscope according to claim 2, wherein the silicon material is peroxide cross-linked.

5. Endoscope according to any one of claims 1 to 4, wherein the rubber material is extruded into a tube during manufacture.

6. Endoscope according to any one of claims 1 to 5, wherein the everting tube is post-treated by heat treatment at 120 to 180 deg. C.

7. Endoscope according to any one of claims 1 to 6, wherein the everting tube is post-treated by heat treatment for 30 to 180 minutes.

8. Endoscope according to any one of claims 1 to 7, wherein the everting tube is treated by washing at 30 to 90 deg. C.

## Revendications

1. Endoscope comportant une tige d'endoscope, qui est fabriquée à partir d'un matériau siliconé extrudé, et un entraînement par manchon inversé, constitué d'un manchon inversé qui entoure avec glissement possible et avec un mouvement relatif la tige de l'endoscope pour transférer une force de propulsion, le manchon inversé étant constitué d'un matériau de caoutchouc extrudé, le manchon inversé étant soumis à un post-traitement par trempe et/ou lavage, **caractérisé en ce que** la tige de l'endoscope comprend, sur sa surface périphérique, un motif continu en relief, courant en spirale, qui a été produit lors de l'extrusion de la tige de l'endoscope, et qui définit, en tant qu'élément d'écartement entre la tige de l'endoscope et le manchon inversé qui l'entoure, un écart dans lequel est un introduit un lubrifiant, le lubrifiant étant aussi incorporé dans la surface du motif en relief, pour émerger de ce dernier sous l'application d'une pression.

2. Endoscope selon la revendication 1, dans lequel le matériau de caoutchouc est choisi parmi un matériau siliconé, un matériau PTFE, un matériau ePTFE et des matériaux plastiques thermoplastiques, de préférence un polyuréthanne thermoplastique dans lequel de préférence une huile est incorporée.

3. Endoscope selon la revendication 2, dans lequel le matériau siliconé est réticulé par addition d'un composé du platine.

4. Endoscope selon la revendication 2, dans lequel le matériau siliconé a subi une réticulation de type peroxydique.

5. Endoscope selon l'une des revendications 1 à 4, dans lequel le matériau de caoutchouc est extrudé en un manchon lors de la fabrication.

6. Endoscope selon l'une des revendications 1 à 5, dans lequel le manchon inversé subit un post-traitement par une trempe à 120 à 180 degrés C.

7. Endoscope selon l'une des revendications 1 à 6, dans lequel le manchon inversé subit un post-traitement par trompe pendant 30 à 180 minutes.

8. Endoscope selon l'une des revendications 1 à 7, dans lequel le manchon inversé subit un post-traitement par lavage à 30 à 90 degrés C.
